# EUROPEAN PATENT APPLICATION

(11) **EP 4 534 098 A1**
(43) Date of publication of application: **09.04.2025**
(21) Application number: 23816164.0
(22) Date of filing: 02.06.2023
(51) Int. Cl.: A61K 39/00, A61K 39/39, A61K 48/00, A61P 11/00, A61P 13/12, A61P 31/20, A61P 37/04, A61P 17/00, A61K 9/19, C12N 7/01, C12N 15/34, C12N 15/86, C12N 15/866, A61K 38/02, A61K 35/64

(54) **ORAL VACCINE COMPOSITION**

(30) Priority: 02.06.2022 JP 2022090367; 11.01.2023 JP 2023002630
(71) Applicant: Kaico Ltd., Fukuoka-shi, Fukuoka 819-0388 (JP); Kyushu University, National University Corporation, Nishi-ku Fukuoka-shi Fukuoka 819-0395 (JP)
(72) Inventor: YAMATO, Kenta, Fukuoka-shi, Fukuoka 819-0388 (JP); TANIGUCHI, Masahiro, Fukuoka-shi, Fukuoka 819-0388 (JP); NAKATAKE, Hirokazu, Fukuoka-shi, Fukuoka 819-0388 (JP); ESAKI, Keiichi, Fukuoka-shi, Fukuoka 819-0388 (JP); KUSAKABE, Takahiro, Fukuoka-shi, Fukuoka 819-0395 (JP)
(74) Representative: J A Kemp LLP
(86) International application number: PCT/JP2023/020590
(87) International publication number: WO 2023/234407

(57) **Abstract**

An oral vaccine composition against porcine circovirus associated diseases, comprising the pupa or cell of a baculovirus infectious insect, which is subjected to an infection treatment with a recombinant baculovirus, into which porcine circovirus type 2 protein-encoding DNA is introduced, and a freeze-drying treatment.

## Description

### Technical Field

The present invention relates to an oral vaccine composition against porcine circovirus associated diseases, and a method for producing the same.

### Background Art

Porcine circovirus associated diseases are diseases caused by porcine circovirus type 2 (PCV2). These diseases are most commonly developed when pigs are at 7 to 16 weeks of age, and symptoms such as loss of energy, stunted growth, emaciation, dyspnea, swollen lymph nodes on the body surface, jaundice, and miscarriage/stillbirth are observed. Thus, it is necessary for pig producers and veterinarians to develop an effective preventive or therapeutic method against porcine circovirus-related disease.

By the way, baculovirus is a nucleopolyhedrovirus (NPV) that infects insects as its main hosts, and forms a protein having a crystalline structure called polyhedrin in the nucleus of infected cells during the multiplication process. Thus, as one of methods for producing a protein of interest using a baculovirus-silkworm system, there is a method comprising introducing a gene encoding the protein of interest into a baculovirus, and then inoculating the recombinant baculovirus into silkworm larvae or pupae to allow the silkworm to produce the protein of interest (Non Patent Literature 1). Thus, when a protein is produced using a baculovirus-silkworm system, it is useful in that the protein of interest can be produced in a large amount.

### Citation List

### Non Patent Literature

Non Patent Literature 1: Maeda et al., "Production of human α-interferon in silkworm using a baculovirus vector." Nature, 315, 592-594 (1985)

### Summary of Invention

### Technical Problem

Under the above-described background, it is desired to develop a method for simply producing an oral vaccine against porcine circovirus-related disease in a large amount, using a baculovirus-silkworm system.

### Solution to Problem

The present inventors have found that a recombinant baculovirus, into which PCV2 protein-encoding DNA has been introduced, is infected into a silkworm pupa, which is then freeze-dried, so that immunogenicity can be surprisingly maintained, thereby completing the present invention.

Specifically, the present invention is, for example, as follows.
[1] An oral vaccine composition against porcine circovirus associated diseases, comprising the pupa or cell of a baculovirus infectious insect, which is subjected to an infection treatment with a recombinant baculovirus, into which porcine circovirus type 2 protein-encoding DNA is introduced, and a freeze-drying treatment.
[2] The oral vaccine composition according to the above [1], wherein the porcine circovirus type 2 protein-encoding DNA is as set forth in SEQ ID No: 1.
[3] The oral vaccine composition according to the above [1], further comprising a solution containing an adjuvant.
[4] The oral vaccine composition according to the above [1], which comprises at least 0.001% by weight of the pupa with respect to the total weight of the composition.
[5] The oral vaccine composition according to the above [1], wherein the porcine circovirus-related disease is at least one selected from the group consisting of postweaning multisystemic wasting syndrome, porcine dermatitis and nephropathy syndrome, porcine respiratory disease complex, and reproductive disorder.
[6] A method for producing an oral vaccine against porcine circovirus associated diseases, comprising a step of allowing a recombinant baculovirus, into which porcine circovirus type 2 protein-encoding DNA is introduced, to infect into the larva or pupa of a baculovirus infectious insect, and freeze-drying a pupa formed from the larva after the infection, or the pupa after the infection.
[7] A method for producing an oral vaccine against porcine circovirus associated diseases, comprising a step of allowing a recombinant baculovirus, into which porcine circovirus type 2 protein-encoding DNA is introduced, to infect into baculovirus infectious cells, and freeze-drying the cells after the infection.
[8] The oral vaccine composition according to the above [6] or [7], wherein the porcine circovirus type 2 protein-encoding DNA is as set forth in SEQ ID No: 1.
[9] The method according to the above [6], wherein the insect is a silkworm.
[10] The method according to the above [7], wherein the cells are derived from *Spilosoma imparilis, Antheraea pernyi, Bombyx mori* (silkworm), *Spodoptera frugiperda,* or *Trichoplusia ni.*
[11] A method for immunizing a pig against a porcine circovirus, comprising a step of administering the composition according to any one of the above [1] to [5] to the pig.

### Advantageous Effects of Invention

According to the present invention, an oral vaccine composition against porcine circovirus associated diseases and a method for producing the same are provided. Since the produced vaccine composition for oral administration can be directly used in administration, the vaccine composition can be comprised in, for example, pig feed, etc.

### Brief Description of Drawings

[Figure 1] Figure 1 is a view showing challenge test design.
[Figure 2] Figure 2 is a view showing a transition in the PCV2 viral genome copy number in serum.
[Figure 3] Figure 3 is a view showing a transition in the PCV2 viral genome copy number in organs.
[Figure 4] Figure 4 is a view showing a transition in the body weight change.
[Figure 5] Figure 5 is a view showing the antibody titers of IgG and IgA in the colostrum of sows at the time of farrowing.
[Figure 6] Figure 6 is a view showing the antibody titers of IgG and IgA in the serum of piglets at 1 day of age.
[Figure 7] Figure 7 is a view showing a comparison of the amount of body weight increased from virus challenge to 4 weeks after the virus challenge (at the end of the test).
[Figure 8] Figure 8 is a view showing a transition over time in the PCV2 gene level after virus challenge.
[Figure 9] Figure 9 is a view showing a transition in the amount of an antigen-specific IgG antibody from initiation of immunization to before virus challenge.
[Figure 10] Figure 10 is a view showing a transition over time in the response of an antigen-specific IgG antibody.

### Description of Embodiments

### 1. Outline

When a vaccine antigens against an infectious disease is produced using a silkworm heterologous protein expression system, a recombinant baculovirus, into which a vaccine antigen gene derived from a target pathogenic microorganism (a virus, etc.) has been inserted, is produced, and this recombinant baculovirus is then inoculated into a silkworm larva or pupa, so that the virus multiplies in the silkworm body to produce the vaccine antigen. In the case of a vaccine that is to be administered by ordinary injection, it is necessary to perform certain purification of the vaccine antigen protein. It is considered that the purification requires a combination of several chromatograms, such as affinity purification, ion exchange purification, and ammonium sulfate precipitation. However, if a silkworm pupa itself, in which a vaccine antigen is expressed, can be administered as a vaccine antigen, the cost of purification can be reduced, and the labor involved in injection administration can be expected to be reduced.

In the present invention, DNA encoding a porcine circovirus type 2 (PCV2) protein has been introduced into a pupa or a cell of a baculovirus infectious insect, and the pupa or cell of the DNA-introduced recombinant baculovirus infectious insect has been subjected to a freeze-drying treatment, so that the pupa and the cell, which have been ensured to have high antigenicity, have been successfully acquired. Then, pigs have been fed with these pupae or cells, and whether antibody production response is actually enhanced has been verified. As a result, a significant increase in the antibody production response to a PCV2 antigen was observed.

The present invention is based on the above-described findings.

The present invention provides an oral vaccine composition against porcine circovirus associated diseases, comprising the pupa or cell of a baculovirus infectious insect, which is subjected to an infection treatment with a recombinant baculovirus, into which PCV2 protein-encoding DNA is introduced, and a freeze-drying treatment. The recombinant baculovirus involves an infection treatment, freeze-drying, and also, a combination of the two treatments.

Moreover, the present invention provides a method for producing an oral vaccine against porcine circovirus associated diseases, comprising a step of allowing a recombinant baculovirus, into which PCV2 protein-encoding DNA is introduced, to infect into the larva or pupa of a baculovirus infectious insect, and freeze-drying a pupa formed from the larva after the infection, or the pupa after the infection.

Furthermore, the present invention provides a method for producing an oral vaccine against porcine circovirus associated diseases, comprising a step of allowing a recombinant baculovirus, into which PCV2 protein-encoding DNA is introduced, to infect into baculovirus infectious cells, and freeze-drying the cells after the infection.

### 2. Recombinant baculovirus, into which PCV2 protein-encoding DNA is introduced

In the present description, examples of the antigen may include circovirus antigens such as, for example, a porcine circovirus antigen (PCV). More specific examples of the antigenic protein may include ORF2(1-233) of a porcine circovirus type 2 (PCV2) isolated strain PCV2a (GenBank accession #AF055392), and a PCV2 KU08-1 strain (PCV2a: GenBank Accession No. LC381288). and PCV2 KU08-1 strain (PCV2a: GenBank Accession No. LC381288). In the present invention, the nucleotide sequences of the DNAs listed in the above-described accession numbers can be optimized to the silkworm codon. The nucleotide sequence obtained by optimization of the nucleotide sequence of Accession No. LC381288 is shown in SEQ ID No: 1.

The size of an antigen is not particularly limited, and it may be, for example, 5 to 1000 kDa, 10 to 500 kDa, or 30 to 200 kDa.

Immunogenicity means the property of an antigen to induce antibody production or cellular immunity. The presence or absence of immunogenicity can be evaluated, for example, by the presence or absence of antibody production after antibody administration. The expression of an antibody can be confirmed by methods known to those skilled in the art, such as enzyme-linked immunosorbent assay (ELISA), Western blotting, immunoprecipitation, flow cytometry, and immunohistochemical staining.

The method of introducing PCV2 protein-encoding DNA into a baculovirus can be carried out by methods known to those skilled in the art, for example, by the methods described in Maeda et al., Nature 315, 592-594 (1985); Y. Matsuura et al., Virology, (1989) 173, 674-682; etc. For instance, the above-described DNA (including cDNA) is cloned and is incorporated into a baculovirus transfer vector to obtain a recombinant baculovirus transfer vector. Then, using this recombinant baculovirus transfer vector, recombinant baculovirus DNA is obtained by a homologous recombination method or a transposon transfer method. This recombinant baculovirus DNA is introduced into insect cultured cells by a known method such as a lipofection method to obtain a recombinant baculovirus.

The baculovirus transfer vector is obtained by subcloning a DNA fragment containing the polyhedrin gene of baculovirus genomic DNA into a plasmid. Such a baculovirus transfer vector can be prepared by a known method, or a commercially available vector can also be used. Examples of the commercially available vector may include pAcYM1, pAcG2T, pAcGP67 and VL1392 (all of which are manufactured by Pharmingen), and pDEST8 (Invitrogen).

In the present invention, examples of the types of a baculoviruses used to produce a recombinant baculovirus may include *Autographa californica* multiple nucleopolyhedrovirus (AcNPV), *Bombyx mori* nucleopolyhedrovirus (BmNPV), *Orgyia pseudotuberculosis* multiple nucleopolyhedrovirus (OpNPV), and *Lymantria disper* multiple nucleopolyhedrovirus (LdNPV). Among these, *Bombyx mori* nucleopolyhedrovirus (BmNPV) is preferable.

### 3. Infection into larva or pupa of baculovirus infectious insect

In the present invention, a recombinant baculovirus is infected into the larva or pupa of a baculovirus infectious insect serving as a host, or into baculovirus-infected cells serving as hosts (which are collectively referred to as a "host").

The insect serving as a host may be a lepidopteran insect, and the host insect is not particularly limited as long as the insect is suitable for protein expression and has baculovirus infectivity. Examples of the host insect may include *Spilosoma imparilis, Antheraea pernyi, Bombyx mori* (silkworm), and *Spodoptera frugiperda.* The insect may have either a pupa or larva form.

In addition, the cells serving as hosts are not particularly limited, as long as they are suitable for protein expression and are of a baculovirus infectious cell line. Herein, "baculovirus infectious" cells mean that baculovirus can be infected into the cells, and one of the characteristics of cells having baculovirus infectivity is the expression of a glycoprotein called GP64 on the cell surface. Accordingly, as long as the cells have such a property, the type of the cells is not limited. Examples of the cells may include the cultured cells of insect cells or other cells.

Examples of the insect cells are as follows.
Spilosoma imparilis-derived cells: SpIm
Antheraea pernyi-derived cells: Anpe
*Bombyx mori* (silkworm)-derived cells: BmN, BmN4, Oyanagi-2, and Bme21
Spodoptera frugiperda-derived cells: Sf9 and Sf21

The method of allowing a recombinant baculovirus to infect into an insect or a cell, which serves as a host, can be carried out by a method known to those skilled in the art. For example, the recombinant baculovirus obtained in the above-described step is injected into a pupa or a larva. In the case of allowing the recombinant baculovirus to infect into host cells, a solution containing the recombinant baculovirus may be added to a cell culture medium.

When host insects or host cells are infected with the recombinant baculovirus and are bred or cultured for 1 to 9 days, antigenic proteins are expressed in the host insects or the host cells.

Herein, in the case of production of a protein of interest using the conventional baculovirus-silkworm system, after the protein of interest is expressed in the body of a silkworm pupa or larva infected with the recombinant baculovirus, the pupa is crushed and suspended, or a body fluid is recovered from the larvae, and various types of purification are carried out, so that the protein of interest is purified. In contrast, according to the method of the present invention, such extraction and/or purification treatments of the protein of interest are not required. A pupa or a cell, in which a PCV2 protein is expressed, can be subjected to a freeze-drying treatment, and then, can be directly used.

### 4. Freeze-drying of host infected with recombinant baculovirus

The timing of freeze-drying the pupa or the cell serving as a host, which has been infected with the recombinant baculovirus, is preferably after a sufficient amount of PCV2 protein is expressed in the host (e.g. in the body of a silkworm pupa). On the other hand, when a larva is infected with the baculovirus, the larva is allowed to grow until it becomes a pupa. Therefore, the timing of freeze-drying is after pupation if the infected silkworm is a larva. If the infected silkworm is a pupa, it can be freeze-dried at any period after infection. The freeze-drying may be performed without cutting or crushing the pupa infected with the recombinant baculovirus, while it is still in the form of a pupa. Otherwise, the freeze-drying may also be performed after cutting or crushing the pupa.

In the present description, the expressions "as is" and "in the form of a pupa" mean that the silkworm is used or included substantially in the form of a pupa, etc. For example, even if unintentionally a part of the pupa is lost in the production process, the pupa may be determined to be in the form of a pupa if 90% or more, 80% or more, 70% or more, 60% or more, or 50% or more of the pupal shape is maintained.

Freeze-drying is a method of drying a pupa under reduced pressure (e.g., maintained in a vacuum) in a frozen state. Freeze-drying can be performed by methods known to those skilled in the art, for example, freeze-drying can be carried out using a commercially available freeze-dryer. The temperature for the freeze treatment can be appropriately set by those skilled in the art, and the freeze treatment temperature is, for example, -90°C to -5°C, -80°C to -10°C, or -50°C to -10°C. In addition, the reduced pressure conditions can also be appropriately adjusted by those skilled in the art, and the pressure is set to be, for example, 2 Pa to 20 Pa, 3 Pa to 20 Pa, or 10 Pa to 20 Pa. The freeze-drying time is, for example, from 1 hour to 168 hours, and preferably from 8 hours to 96 hours. In an aspect of the present invention, the freeze-drying time is, for example, 8 to 36 hours.

As a result of freeze-dying, most of the water is removed from the pupae or the cells, and they become a dry state. The water content in the pupae after the freeze-drying may be, for example, 1% by weight or less, 0.5% by weight or less, 0.1% by weight or less, 0.01% by weight or less, or 0.001% by weight or less, with respect to the total weight of the pupae or the cells. From the viewpoint of facilitating the maintenance of immunogenicity, the closer the water content in the pupae is to 0% by weight, more preferable it is.

Herein, in the present invention, a pupa or a cell can be optionally subjected to a liquid agent treatment, an immersion treatment, a light treatment, a gas treatment, a plasma treatment, a heat treatment, or a pressurized heat treatment. The timing of performing such a liquid agent treatment, an immersion treatment, a light treatment, a gas treatment, a plasma treatment, a heat treatment, or a pressurized heating can be before or after freeze-drying, and it is preferably, for example, after the pupa or the cell is sufficiently dried by freeze-drying. The step of freeze-drying a host infected with a recombinant baculovirus and the step of performing a liquid agent treatment, an immersion treatment, a light treatment, a gas treatment, a plasma treatment, a heat treatment, or a pressurized heat treatment can be carried out consecutively. In other words, after the freeze-drying, such a liquid agent treatment, an immersion treatment, a light treatment, a gas treatment, a plasma treatment, a heat treatment, or a pressurized heat treatment can also be carried out without any other treatment steps. The timing after freeze-drying can be adjusted, as appropriate, according to the conditions of the dried silkworm pupa, etc., and the step of performing a liquid agent treatment, etc. is, for example, within 1 to 24 hours after the freeze-drying, such as within 24 hours, within 12 hours, within 3 hours, or within 1 hour. It is preferable to perform a liquid agent treatment, an immersion treatment, a light treatment, a gas treatment, a plasma treatment, a heat treatment, or a pressurized heat treatment on the freeze-dried pupa as in the form of a pupa, without cutting or crushing it.

The pupa and cell prepared by the present production method have immunogenicity. Therefore, in the present invention, a PCV2 protein does not need to be extracted, and can be directly used for oral administration.

Furthermore, in one aspect of the present invention, since the freeze-dried pupa is dried and is in a sponge-like state, a liquid can easily penetrate into the pupa by immersing it in the liquid. Accordingly, the production method according to the present embodiment may further comprise a step of immersing a freeze-dried pupa or a pupa that has been subjected to a pressurized heat treatment as necessary, in a liquid. As a liquid to be impregnated, it is preferable to use a solution containing an adjuvant, since it can easily improve the effects as a vaccine. By immersing the freeze-dried pupa in a solution containing an adjuvant, the solution containing an adjuvant can easily penetrate into the inside of the pupa. Since cultured cells are converted to a powdered composition by freeze-drying, in the case of using cells, the freeze-dried cells may be immersed in a solution containing an adjuvant. Otherwise, the pupa in the present production method may be mixed with other solids or liquids. For example, it is preferable to use a solid containing an adjuvant.

The pupa or the cell produced by the present invention may be used as it is, or may also be used after it has been cut, crushed, liquefied. Otherwise, after a crude extract has been recovered from the pupa, the pupa may also be re-impregnated with the crude extract. Or, as mentioned above, the pupa may be used by being infiltrated with a liquid, or may also be used by being mixed with other solids.

Examples of the form, in which the pupa is directly used, may include a form, in which the pupa is directly administered as a vaccine via oral administration, and a form, in which the pupa is directly ingested as an edible use.

Examples of the form, in which a cut or crushed pupa is used, may include a form, in which a cut or crushed pupa is administered as it is, a form, in which a crushed pupa is mixed with other materials that can be orally administered and is administered, and a form, in which a crushed pupa is mixed with a feed and is ingested as an edible use. For example, in order to adjust the dose in consideration of the immunogenicity of the pupa, the pupa may be cut into, for example, 1/2, 1/3, 1/4, and so on. The degree of crushing is not particularly limited, as far as the crushed pupa exhibits immunogenicity. For example, the pupa may be crushed into a powder state. Cutting can be carried out using, for example, food scissors and scissors for laboratory animals, whereas crushing can be carried out using, for example, a mixer, a hand mill, and a pulverizer. In the case of using cells, the cells can be treated in the same manner as when the pupa is crushed.

Examples of the oral administration may include intraoral administration and sublingual administration. In the present description, administration also includes a case where a pig ingests the pupa by itself. When the pupa is administered as in the form of a pupa, intraoral administration is preferable, and the pupa may be included in a feed, so that the pig eats it by itself.

Examples of an orally administrable additive may include a food material, a beverage material, an excipient, a thickener, a stabilizer, an antiseptic, a pH adjuster, a sweetener, a colorant, an emulsifier, a flavoring agent, and pharmaceutical additives described later. The pupa or the cell is mixed with a food material or a beverage material, and it can also be administered as a functional food or a beverage. A Liquid containing an orally administrable material is not particularly limited, as long as the pupa for oral administration has immunogenicity and the liquid is a liquid suitable for oral administration. The liquid may be, for example, water or an aqueous solution.

### 5. Oral vaccine composition

Since the pupa or cell obtained as described above contains a PCV2 protein and has immunogenicity, it is used as an oral vaccine composition.

The diseases, to which the oral vaccine of the present invention is applied, are porcine circovirus associated diseases. The porcine circovirus associated diseases include postweaning multisystemic wasting syndrome (PMWS), porcine dermatitis and nephropathy syndrome (PDNS), porcine respiratory disease complex (PRDC), reproductive disorder, body weight reduction, an increase in mortality, and digestive tract diseases such as diarrhea, and one or several of these diseases can be targeted.

The oral vaccine composition can be processed in any dosage form, such as, for example, a liquid agent (syrup, jelly, etc.), a powder, a granule, a tablet, a powdery agent, a capsule, a mashed feed, a pellet feed, a crumble feed, an expander feed, and a flake feed, as well as in its original pupal shape. Also, when the cell is used as an oral vaccine composition, it can be processed into any dosage form such as a powder, a granule, a tablet, a powdery agent, a capsule, a mashed feed, a pellet feed, a crumble feed, an expander feed, and a flake feed.

The above-described dosage forms can be formulated together with pharmaceutical additives according to the methods usually practiced in the present technical field. Examples of the pharmaceutical additives may include an adjuvant, a carrier for oral administration, a diluent, an excipient, a disintegrant, a binder, a lubricant, a fluidizer, a coating agent, a solubilizing agent, a solubilizer, a thickener, a dispersant, a stabilizer, an antiseptic, a pH adjuster, a tension regulator, a wetting agent, a sweetener, and a flavoring agent. From the viewpoint of improving the effects as a vaccine, the pharmaceutical additive preferably contains an adjuvant.

Examples of the adjuvant may include a Freund's complete adjuvant, a Freund's incomplete adjuvant, a pertussis adjuvant, a Ribi adjuvant, Lipid-A, liposome, aluminum hydroxide, silica, enterotoxin (cholera toxin, cholera toxin α-subunit, and cholera toxin β-subunit), Toll-like receptor (TLR) ligands (Poly(I:C), lipopolysaccharide, flagellin, and CpG), mucoadhesive agents (chitosan and lectin), cytokines, an artificial synthetic adjuvant, an oil adjuvant, and polysaccharides.

In the present invention, a step of mixing a pupa or a cell having immunogenicity with pharmaceutical additives or a liquid containing the pharmaceutical additives may be further included. In the oral vaccine composition of the present invention, the expressed PCV protein is contained in the pupa or the cell. Thus, the component of the pupa or cell having immunogenicity (e.g. freeze-dried pupal powders) may be comprised in an amount of, for example, 0.001% by weight or more, 0.01% by weight or more, 0.1% by weight or more, 1% by weight or more, 2% by weight or more, 5% by weight or more, 10% by weight or more, 20% by weight or more, 30% by weight or more, 40% by weight or more, 50% by weight or more, 60% by weight or more, 70% by weight or more, 80% by weight or more, or 90% by weight or more, with respect to the total weight of the vaccine composition.

The liquid used as a pharmaceutical additive is particularly limited, as long as the oral vaccine composition has immunogenicity and the liquid is pharmacologically suitable for oral administration. Examples of the liquid used as a pharmaceutical additive may include water, and aqueous solutions such as a normal saline or a phosphate-buffered saline (PBS).

Pupa having immunogenicity may be used as it is, or may be cut into pieces, or may be crushed, or may be liquefied, or a crude extract may be recovered from a pupa followed by impregnation of the pupa with the crude extract. In the case of using cells as well, the cells can be crushed.

In the present invention, when the pupa is used as in the form of a pupa, a step of mixing the pupa with a liquid containing pharmaceutical additives may include a step of immersing the pupa after freeze-drying in a liquid. For example, by immersing the pupa in a solution containing an adjuvant, the solution containing an adjuvant can easily penetrate into the pupa. The pupae that have been penetrated by the liquid may be further cut or crushed to prepare an oral vaccine composition. In the case of cultured cells as well, the cultured cells can be used in the same way as a crushed pupa.

When a cut or crushed pupa is used, a step of mixing the pupa with a liquid containing pharmaceutical additives may include a step of dispersing the cut or crushed pupa in the liquid.

The subject, to which the vaccine composition of the present invention is administered, is a pig, and the breed of the pig is not particularly limited. Examples of the breed may include Middle Yorkshire, Large Yorkshire, Berkshire, Landrace, Duroc, and Hampshire.

The vaccine for oral administration of the present invention exhibits immunogenicity, even if it is orally administered as in the form of a pupa. The vaccine may be administered after it is cut or crushed, or as mentioned above, it may be impregnated with a liquid and may be then used.

Examples of the form to be directly administered in a pupal shape may include a form in which a pupa for oral administration is directly orally administered as a vaccine, and a form in which a pupa for oral administration is directly ingested as edible use.

Example of the form in which the pupa is administered by cutting or crushing it (including crushed cells) may include a form in which a cut or crushed pupa or cell is orally administered as it is, a form in which a cut or crushed pupa or cell is mixed with another orally administrable material and the mixture is administered, and a form in which a crushed pupa is mixed with a feed and the mixture is ingested as edible use. For example, a pupa may be cut into 1/2, 1/3, 1/4, etc., in order to adjust the dose in consideration of the immunogenicity of the pupa. The degree of crushing is not particularly limited, as long as the pupa has immunogenicity, and the pupa may be crushed into, for example, powders.

The dose can be set, as appropriate, taking into consideration the administration target and the amount of an antigenic protein expressed in the pupa or the cell, etc. For example, 0.01 to 100 pupae (0.001 g to 30 g) may be administered per dose, or 0.1 to 10 pupae (0.01 g to 3 g) may be administered per dose. The amount of a PCV2 protein expressed is 0.2 mg to 10 mg with respect to about 800 mg of pupa weight, which can be 0.025% by weight to 1.25% by weight per pupa. According to this, the PCV2 protein is contained in one pupa for oral administration, in an amount of 10 times or more the single dose of the PCV2 protein for an ordinary injectable vaccine.

The number of doses and the period of administration can also be set, as appropriate, taking into consideration the administration target and the amount of the PCV2 protein expressed in the pupa or the cell, etc. For example, it may be administered once to five times per day, once to three times per day, or once per day. In addition, the vaccine may be administered, for example, from 1 to 7 days per week, from 1 to 5 days per week, or from 1 to 4 days per week. Moreover, these doses may be repeated for, for example, 2 to 24 weeks, 1 to 7 weeks, 2 to 5 weeks, or 2 to 4 weeks.

The age of pigs at the time of vaccine administration is not particularly limited, and it may be any age from piglets to adult pigs. It is to be noted that adult pigs include pregnant sows and gilts.

Upon vaccine administration, pigs may be fasted for approximately 1 to 3 hours before administration (before ingestion).

Specifically, for example, the vaccine can be administered to pigs as follows. Dose: 0.1 to 5 pupae (0.01 g to 1.5 g) is administered once a day (ingestion).

### Period: 1 to 5 doses per week, repeated for 3 to 4 weeks

Furthermore, additional immunization may be performed thereafter, by administering similar doses, for example, every 1 to 4 weeks, preferably every 1 to 2 weeks.

Immunity of pigs against porcine circovirus-related disease can be caused by administering the composition of the present invention to the pigs.

The vaccine composition of the present invention can also be used in combination with or by combining with other vaccines. Such other vaccines may have immunogenicity against the PCV2 protein or may have immunogenicity against different antigenic proteins. If other vaccines have immunogenicity against the same antigenic protein as that of the pupa or the cell, for example, the pupa of the present invention may be administered for additional immunization, etc. of the other vaccines mentioned above.

Further, in the present invention, IgG and IgA responsive reactions are observed in the colostrum of pregnant sows that have ingested the vaccine composition of the present invention. This indicates that the effects of the vaccine can be obtained even in breast milk. In addition, when piglets received the colostrum of their sow, IgG and IgA responsive reactions were unexpectedly observed in the serum of 1-day-old piglets. This indicates that the effects of the vaccine are inherited by piglets via breast milk.

### Examples

Hereinafter, the present invention will be more specifically described in the following examples. However, these examples are not intended to limit the technical scope of the present invention.

### [Example 1] Confirmation test of effects of oral administration of novel vaccine against PCV2 to piglets -1

### < Materials and Methods >

### (1) Silkworm cultured cells and strain

Silkworm culture cells were prepared according to the method described in the previous reference (Masuda et al., 2018). As a silkworm pupae strain, Kinshu Showa (Atsumaru HD, Kumamoto, Japan) was used.

### (2) Preparation of recombinant baculovirus

A recombinant baculovirus was prepared according to the method described in the previous reference (Masuda et al., 2018). Specifically, as a genotype of PCV2, PCV2 KU08-1 strain (PCV2a; Kagoshima University) (GenBank accession number: LC381288) was used. The region corresponding to the entire length of PCV2 ORF2 (the amino acids at positions 1 to 233) was optimized to the silkworm codon, and DNA having the optimized nucleotide sequence (SEQ ID No: 1) was then synthesized. The synthesized DNA was amplified and was then subcloned into a pENTR11 vector to prepare pDEST8-PCV2 ORF2 (1-233). The silkworm codon-optimized nucleotide sequence is shown in SEQ ID No: 1 (see below), and the amino acid sequence encoded by the nucleotide sequence is shown in SEQ ID No: 2.

ATGGGCACTTATCCGAGGAGGCGTTACCGCAGAAGAAGACACAGACCCCGTA
GTCACCTGGGACAAATACTCAGAAGGAGACCGTGGCTGGTCCACCCGCGTCA
TAGGTATAGATGGAGACGCAAAAACGGAATCTTCAATACGAGGCTGAGCAGA
ACTTTTGGCTACACCGTCAAGGCAACAACTGTACGCACTCCGTCCTGGGCGGT
CGACATGATGCGTTTCAACATTGACGATTTTGTACCTCCAGGTGGAGGCACCA
ACAAAATCTCGATTCCCTTCGAATACTATCGCATTCGTAAAGTGAAGGTTGAGT
TTTGGCCTTGCTCGCCAATAACGCAAGGTGACCGTGGTGTGGGAAGTACAGC
TGTTATCCTGGACGATAACTTCGTGACCAAGGCTACGGCCTTGACATACGATCC
TTATGTTAATTACTCATCTAGGCACACAATTCCTCAACCATTCAGCTATCATTCC
CGCTACTTTACGCCGAAACCCGTACTCGACTCTACAATCGATTACTTCCAGCCA
AACAATAAGAGAACTCAACTGTGGTTGCGCCTCCAGACCTCACGTAACGTCG
ACCACGTAGGCCTGGGTACTGCCTTTGAAAACTCTATCTACGACCAAGATTAC
AACATCAGGGTGACCATGTACGTTCAGTTCAGAGAGTTCAACCTCAAGGATCC
GCCCCTTAAGCCTTAA

As a recombinant baculovirus, BmNPV-T3ΔPE3P, in which 6 types of genes (*chitinase A, cathepsin, egt, p26, p10 and p74*) were deleted from a BmNPV-T3 strain in order to improve protein expression and to extend the survival period after silkworm infection, was used.

### (3) Expression of PCV2 capsid protein in insect cultured cells and silkworm pupae

Expression of a PCV2 capsid protein was carried out according to the method described in the previous reference (Masuda et al., 2018). Pupae were infected with 1 x 10⁵ plaque-forming units (p.f.u.) of the recombinant baculovirus per pupa, and the infection period was set to be 4 days.

### (4) Preparation of silkworm pupal oral vaccine antigen expressing PCV2 capsid protein

The infected silkworm pupae were cryopreserved at -80°C. Upon preparation of powders, the pupae were removed from the condition of -80°C, and were then pulverized using a mill mixer (Iwatani Corporation, Osaka).

### (5) Preparation of PCV2 virus

PCV2 virus was isolated and identified from the sera (the number of PCV2 genes: approximately 5 x 10⁵ copies/µL) of 10- to 12-week-old pigs (several samples), which had been confirmed to have persistent PCV2 viremia and fecal excretion of the virus, and thereafter, the PCV2 virus was prepared as a challenge strain of PCV2 by 3 to 4 passages of culture.

### (6) Immunization schedule and sample collection in pig test

Nine domestic pigs (4 weeks old) (including one reserve pig), which had been previously confirmed to be negative for PCV2 by PCR and ELISA kits, were introduced into the Sanwa Farm of Kyodoken Institute, and were tested.

The domestic pigs were randomly divided into two groups: an oral vaccine administration group and a control (non-administration) group.

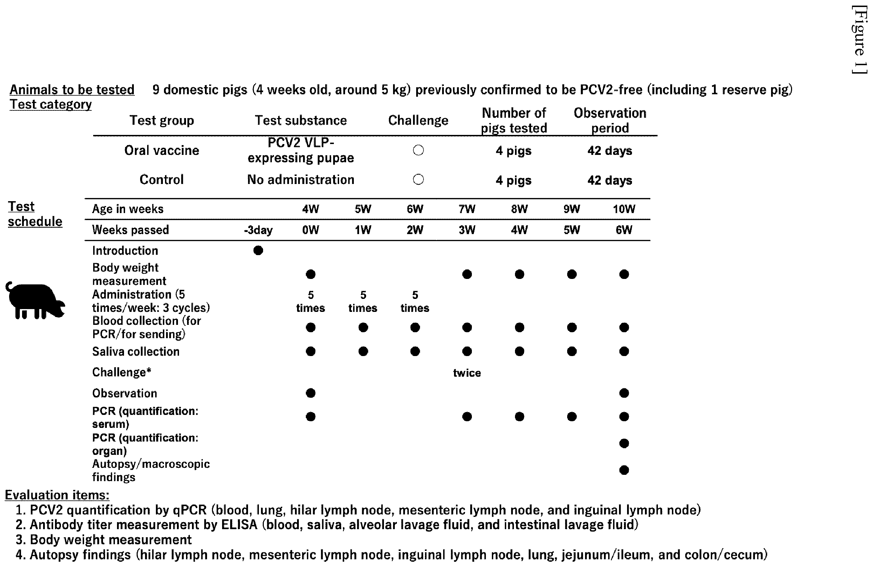

The test schedule is as shown in Figure 1.

1) Administration of test substance
   Starting at 4 weeks of age, 3 days after the introduction, in the oral vaccine administration group, oral administration of the vaccine was carried out by adding the vaccine to a feed for pigs for 3 cycles of 5 doses/7 days. The dose of the novel vaccine was set to be 1 g of silkworm pupal powder per dose. In addition, a non-administration control group was also established. It is to be noted that a total of 8 pigs, 4 pigs in each group, were tested.
2) Challenge method
   At a time point of 21 days (7 weeks old) after initiation of the immunization, the pigs were challenged by spraying 5 mL of porcine PCV2 (KDK field isolate strain: PCV2) culture solution into the nasal cavity of the test pigs once a day for 2 days. The number of challenging bacteria was measured by real-time PCR (qPCR).
3) Breeding of the test pigs
   During the test period, piglets were fed with SDS No. 2 [manufactured by FeedOne Corporation], a standard diet for piglet tests that does not contain antimicrobial substances.
4) Observation period
   The observation period was set to be six weeks after the first administration.
5) Weight measurement
   Body weights were measured once a week, at the induction, at initiation of the first administration (4 weeks old), and at the first challenge (7 weeks old).
6) Quantification of PCV2 viral genome copy number
   The PCV2 viral genome copy number was determined by real-time PCR.
7) Autopsy and lesion observation
   Autopsy was carried out at 21 days after the first challenge.
8) Statistical analysis
   Differences between groups were evaluated by Student t-test for PCR quantification. The significance level was set at 5%.

### < Results >

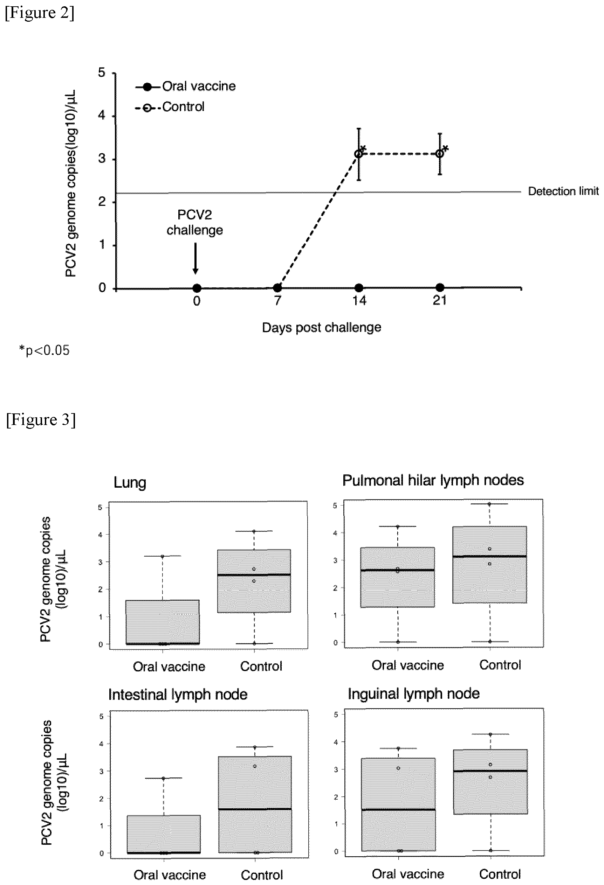

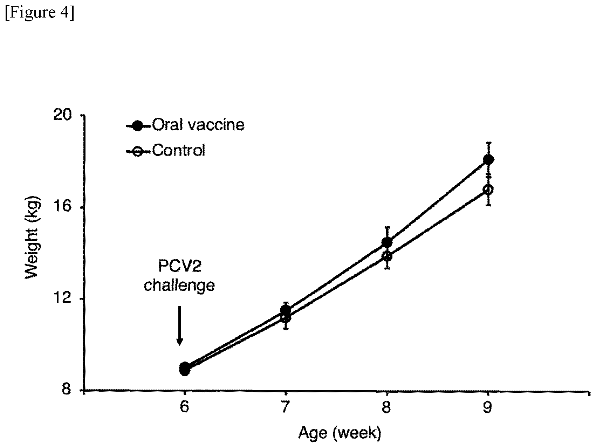

1) Evaluation of PCV2 viral genome copy number in serum (evaluation of viremia)
   In the oral vaccine administration group, the PCV2 viral genome copy number was transitioned to be less than the detection limit even after PCV2 virus infection, whereas in the non-administration group (control group), the PCV2 viral genome was detected after 7 days of infection. In the oral vaccine administration group, proliferation of the PCV2 virus was significantly suppressed (p<0.01) (Figure 2).
2) Evaluation of PCV2 viral genome copy number in each organ
   The PCV2 viral genome copy number in lung, hilar lymph node, mesenteric lymph node, and inguinal lymph node was evaluated, and as a result, it was found that the virus detection rate and the virus amount tended to be lower in the oral vaccine administration group than in the non-administration group, and it showed a trend of lower viral detection rate and viral load in the oral vaccine group than in the non-administration group (Figure 3).
3) Body weight change
   One of the clinical symptoms of PCV2 virus is suppression of body weight gain. The oral vaccine administration group tended to show better weight gain after the PCV2 virus infection than the non-administration group (Figure 4).
4) Observation after organ autopsy
   Lesion severity in the autopsied lymph nodes, lungs, and intestinal tracts was evaluated. As a result, the lesion onset rate and the severity did not significantly differ between the oral vaccine administration group and the non-administration group, but the lesion expression rate in the colon and cecum was lower in the oral vaccine administration group (Table 1).

**[Table 1].**

| Table 1: Observation of lesions in organs | | | | | | | |
|---|---|---|---|---|---|---|---|
| Test group | Animal No. | Lymph node | | | Lung | Intestinal tract | |
| | | Pulmonary hilum | Mesentery | Groin | | Jejunum/Ileum | Colon/Cecum |
| Orally vaccinated | 1 | 0 | 1 | 2 | 0 | 0 | 0 |
| | 2 | 1 | 0 | 0 | 0 | 0 | 0 |
| | 3 | 1 | 0 | 0 | 0 | 0 | 0 |
| | 4 | 2 | 1 | 3 | 0 | 0 | 3 |
| Unvaccinated | 5 | 1 | 1 | 0 | 0 | 0 | 1 |
| | 6 | 1 | 0 | 2 | 0 | 0 | 2 |
| | 7 | 0 | 1 | 0 | 0 | 0 | 0 |
| | 8 | 1 | 1 | 1 | 0 | 0 | 2 |

| | | | | | | | |
|---|---|---|---|---|---|---|---|
| Lymph nodes: Enlargement (0: None, 1: Mild, 2: Moderate, 3: Severe) Lung: Edema-like degeneration (circovirus-infected lung) (0: None, 1: Mild, 2: Moderate, 3: Severe) Intestinal tract: White spots (0: None, 1: Mild, 2: Moderate, 3: Severe) | | | | | | | |

### References

Masuda, A., Lee, J. M., Miyata, T., Sato, T., Hayashi, S., Hino, M., Kusakabe, T. (2018). Purification and characterization of immunogenic recombinant virus-like particles of porcine circovirus type 2 expressed in silkworm pupae. J Gen Virol, 99(7), 917-926. https://doi.org/10.1099/jgv.0.001087

### [Example 2] Vaccination test to pregnant pigs

### < Materials and Methods >

### 1. Test animals

Twelve pregnant pigs (less than 2 months old after mating) having low PCV2 antibody titer were used.

### 2. Test category

The test category was as shown in Table 2 below.

**[Table 2]**

| Table 2 Test group | | | | |
|---|---|---|---|---|
| Test group | Test substance | Administration period | Administration | Number of pigs tested |
| T01 | Novel vaccine | Late pregnancy (approximately 4 weeks before expected farrowing) | 5 times/week | 4 pigs |
| T02 | Novel vaccine | Midterm and late pregnancy (approximately 8 and 4 weeks before expected farrowing) | 5 times/week × 2 cycles | 4 pigs |
| T03 | No administration | None | None | 4 pigs |

The test substance is mixed with a feed and is orally administered (feed additive administration).

The test substance is administered at a rate of 5 times/7 days.

The dose of the novel vaccine is set to be 1.0 g/head of silkworm pupal powders once a day.

### 3. Test schedule

The test schedule was set as shown in Table 3 below.

**[Table 3]**

| Table 3 Schedule | | | | | | | | | |
|---|---|---|---|---|---|---|---|---|---|
| | Pregnancy period | | | | At farrowing | | | | Weaning |
| Weeks passed | After pregnancy confirmation | -8W±1W | -4W±1W | | 0W | | 1d | | 3W |
| Introduction | ○ | | | | | | | | |
| Body weight measurement | ○ | | | | | | | | ○ |
| Administration (5 times/week) | | 5 times _{(T01 only)} | 5 times _{(T01 and T02)} | | | | | | |
| Blood collection (for antibody titer measurement) | | ○ | ○ | | ○ | | ○ | | ○ |
| Saliva collection | | ○ | ○ | | ○ | | | | ○ |
| Colostrum collection | | | | | ○ | | | | |
| Observation | | ○ | | | | | | | ○ |
| Autopsy/macroscopic findings | | | | | | | | | |
| Piglet body weight measurement | | | | | ○ | | | | ○ |
| Piglet blood collection | | | | | 5 pigs/sow | | 5 pigs/sow | | 5 pigs/sow |

| | | | | | | | | | |
|---|---|---|---|---|---|---|---|---|---|
| ∘: Implementation of the items described on the left | | | | | | | | | |

### 4. Test methods

1) Administration of test substance
   The test substance was orally administered to an oral immunization group (T02 group) approximately 8 weeks (±1 week) before expected farrowing after the introduction, and further, to the oral immunization groups (T01 and T02 groups), approximately 4 weeks (±1 week) before expected farrowing, the test substance was orally administered by feed addition at a rate of 5 times/7-day cycle (1 g per pig/once). In addition, a non-administration group (T03 group) was also established. A total of 12 pigs, 4 in each group, were tested.
2) Breeding of test pigs
   During the test period, the test pigs were fed with a normal diet for gestation period and lactation period [manufactured by Chubu Shiryo Co., Ltd. or Feed One Co., Ltd.].
3) Observation period
   The observation period was set to be from the first administration to the time of weaning.

### 5. Observation items

1) Body weight measurement (sows)
   Body weights were measured at the time of induction and weaning.
2) Blood collection and antibody titer measurement (sows)
   Blood was collected, and antibody titers were measured by ELISA, at a total of 4 times, namely, at the time of the first administration, at the second administration, at farrowing, and at weaning.
3) Saliva collection and sending (sows)
   Saliva was collected at a total of 4 times upon blood collection.
4) Colostrum collection (sows)
   Colostrum was collected at the time of farrowing.
5) Clinical observation (sows and piglets)
   During the test period (from 8 weeks before expected farrowing to weaning), general clinical symptoms were observed.
6) Body weight measurement (piglets)
   The body weights of all piglets were measured at the time of farrowing and at the time of weaning.
7) Blood collection and antibody titer measurement (piglets)
   Regarding a total of 5 piglets per belly, blood was collected at a total of twice, once at 1 day of age and once at weaning, antibody titers were measured by ELISA.

### 6. Statistical analysis

Statistical analysis was performed by the Kruskal-Wallis test and the Bonferroni-corrected Wilcoxon rank sum test after confirming determination of the normality by the Shapiro-Wilk test.

### 7. Efficacy evaluation

1) PCV2 antigen-specific IgG and IgA antibody titers in serum
2) PCV2 antigen-specific IgG and IgA antibody titers in saliva
3) PCV2 antigen-specific IgG and IgA antibody titers in colostrum

### < Results >

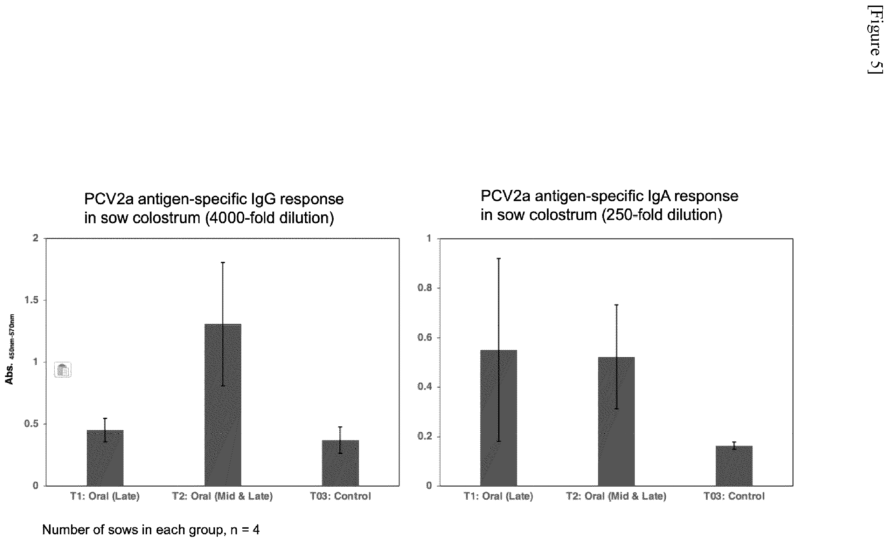

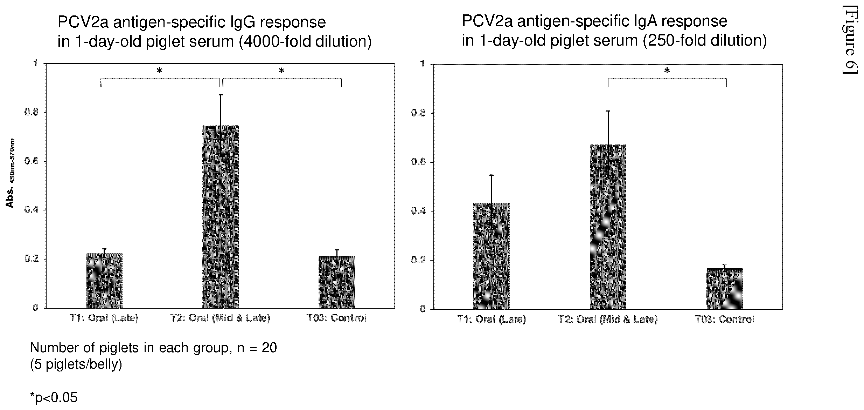

The results are shown in Figure 5 and Figure 6.

Figure 5 is a view showing the antibody titers of IgG and IgA in the colostrum during sow farrowing.

Panel A shows the results of PCV2 antigen-specific IgG response in colostrum (4000-fold dilution) (ELISA). The bars in the graphs T1, T2 and T03 each indicate, from left to right, the absorbance of whey in the oral immunization group (T01 group), the absorbance of whey in the oral immunization group (T02 group), and the absorbance of whey in the non-administration control group (T03 group).

Panel B shows the results of PCV2 antigen-specific IgA responses in colostrum (250-fold dilution) (ELISA). The bars in the graphs T1, T2 and T03 each indicate, from left to right, the absorbance of whey in the oral immunization group (T01 group), the absorbance of whey in the oral immunization group (T02 group), and the absorbance of whey in the non-administration control group (T03 group).

From Figure 5, it was shown that IgG and IgA responsive reactions were observed in the colostrum of sows, indicating that the effects of the vaccine can be obtained even in breast milk.

Figure 6 is a view showing the antibody titers of IgG and IgA in the serum of piglets at the age of 1 day.

Panel A shows the results of the PCV2 antigen-specific IgG response in the serum (4000-fold dilution) of 1-day-old piglets (ELISA). The bars in the graphs T1, T2 and T03 each indicate the absorbance of the serum of piglets farrowed from the oral immunization group (T01 group), the absorbance of the serum of piglets farrowed from the oral immunization group (T02 group), and the absorbance of the serum of piglets farrowed from the non-administration control group (T03 group).

Panel B shows the results of PCV2 antigen-specific IgA response in piglet 1-day-old serum (1000-fold dilution) of 1-day-old piglets (ELISA). The bars in the graphs T1, T2 and T03 each indicate the absorbance of the serum of piglets farrowed from the oral immunization group (T01 group), the absorbance of the serum of piglets farrowed from the oral immunization group (T02 group), and the absorbance of the serum of piglets farrowed from the non-administration control group (T03 group).

From Figure 6, it was shown that IgG and IgA responsive reactions were observed in the serum of piglets at the age of 1 day that received the colostrum of sows inoculated with oral vaccine, indicating that the effects of the vaccine are transferred to the piglets via breast milk.

### [Example 3] Confirmation test of effects of oral administration of novel vaccine against PCV2 to piglets -2 (dosage and administration study).

### < Test Methods >

### 1. Test substance

Novel vaccine: One type of porcine PCV2 novel oral vaccine (PCV2 rCap/VLP expressing silkworm pupal powder)
Positive control: CircoFLEX (manufactured by Boehringer Ingelheim Vetmedica, Inc.)

### 2. Test animals

Piglets farrowed from the negative control group in the new circovirus vaccine test tor breeding pigs, and piglets farrowed from some vaccinated sows, were subjected to the present test, and the efficacy of a novel vaccine for porcine PCV2 infection against the piglet was then confirmed by an experimental challenge test using porcine PCV2 virus. A total of 28 piglets shown in the test category were tested (Table 4).

**[Table 4]**

| **Test category** | | | | | |
|---|---|---|---|---|---|
| **Test Group** | **Test substance to piglets** | **Administration method** | **Dose/number of days** | **Nunber of piglets tested** | **Origin sow** |
| **T01** | **PCV2 Cap-expressing silkworm pupae** | **Oral** | **0.5g x 10days** | **4 piglets** | **Sow test T03** |
| **T02** | **PCV2 Cap-expressing silkworm pupae** | **Oral** | **0.5g x 6days** | **4 piglets** | **Sow test T03** |
| **T03** | **PCV2 Cap-expressing silkworm pupae** | **Oral** | **0.5g x 3days** | **4 piglets** | **Sow test T03** |
| **T04** | **None (maternal antibody only)** | **None** | **None (1g x 5days against a sow)** | **4 piglets** | **Sow test T01** |
| **T05** | **None (maternal antibody only)** | **None** | **None (1g x 10days against a sow)** | **4 piglets** | **Sow test T02** |
| **T06** | **CircoFLEX** | **Intramuscular injection** | **Once** | **4 piglets** | **Sow test T03** |
| **T07** | **None (negative control)** | **None** | **None** | **4 piglets** | **Sow test T03** |

| | | | | | |
|---|---|---|---|---|---|
| **Test substance was administered by forced oral** **administration.** | | | | | |

### 4. Test schedule

The test schedule was as shown in Table 5 below.

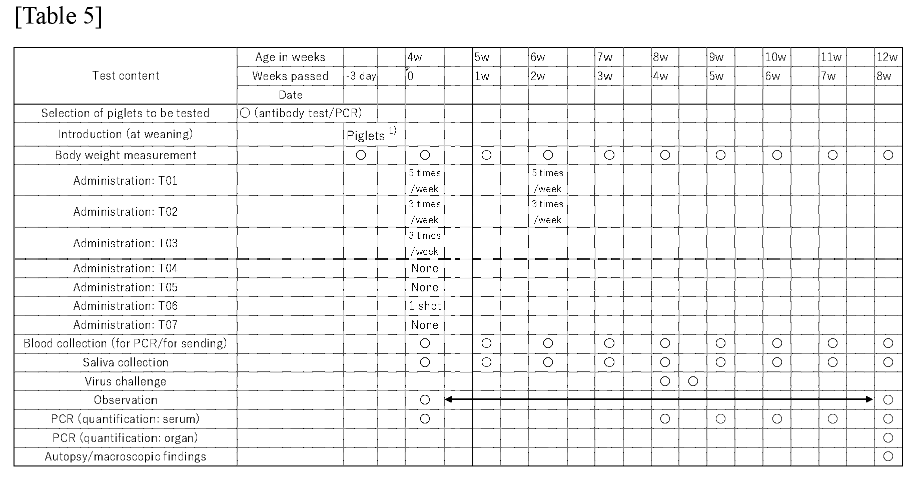

### 5. Test methods

### 1) Administration of test substance

The test substance was administered according to the test category. In the present Example, each group was bred as a group, and the administration was carried out by forced oral administration. For the forced oral administration, 0.5 g of the test substance was weighed in advance, was placed in a 50-mL PP tube, and was orally administered to the pigs after holding them in place. After completion of the oral administration, the pigs were forced to additionally ingest approximately 20 mL of tap water.

There were 7 test groups, and a total of 28 pigs (4 pigs in each group) were tested.

### 2) Challenge method

At a time point of 28 days after initiation of the immunization (from 9 to 10 weeks of age), 5 mL of culture solution of porcine PCV2 (KDK field isolate strain: PCV2) was sprayed into the nasal cavity of the test pigs once a day for 2 days for challenging. It is to be noted that the number of challenging virus was measured by real-time PCR (qPCR).

### 3) Breeding of test pigs

During the test period, piglets were fed with SDS No. 2 [manufactured by Feed One Co., Ltd.], a standard diet for piglet tests that does not contain antimicrobial substances.

### 4) Observation period

The observation period was 8 weeks after the first administration.

### 6. Observation items

1) Body weight measurement
   The pigs were weighed once a week from initiation of the first administration to the time of autopsy.
2) Blood collection, quantification of PCV2 in blood, and antibody titer measurement
   From initiation of the first administration to the time of autopsy, blood was collected a total of 9 times at weekly intervals, and PCV2 quantification (qPCR) in blood was then measured (PCR measurement was performed only after the challenge).
3) Saliva collection and sending
   Saliva was collected upon the blood collection a total of 9 times at weekly intervals from initiation of the first administration to the time of autopsy.
4) Clinical observation
   During the test period (for 56 days), general clinical symptoms were observed. With regard to death and culled cases, the cause was to be investigated and recorded as much as possible, but neither death nor culled cases occurred in the present Example.
5) Autopsy and observation of lung lesions
   Autopsy was performed 28 days after the first challenge. The lung, hilar lymph node, mesenteric lymph node and inguinal lymph node were sampled.
6) PCV2 quantification (real-time PCR)
   Porcine PCV2 was quantified by real-time PCR from sera after the challenge and from organs as investigation targets at autopsy.

### 7. Statistical analysis

1) Body weight gain: Dunnet's test (p<0.05, P<0.01).
2) PCR quantification: Steel-Dwass's method was used to compare the results among the test groups.

### 8. Efficacy evaluation

1) Body weight gain and PCV2 virus amount in blood (q-PCR)
2) IgG antibody titer in serum
3) IgA antibody titer in saliva

### < Test results >

The immunization test was divided into an immunization test for 4 weeks after the setting of the test and a challenge test for the sequent 4 weeks, and was described.

### 1. Immunization test

1) Body weight and body weight gain
   As a result of investigation of body weight and body weight gain in each group, it was found that the average total body weight gain in each group was 18.2 kg to 20.4 kg during the test period for 4 weeks, and as a result of Tukey-Kramer test, no significant difference was confirmed among the groups.
2) Clinical symptoms
   With regard to the clinical symptoms of each group, no abnormalities in the clinical symptoms, such as vitality, appetite, and fecal characteristics, were observed at all during the test period.

### 2. Challenge test

### 1) Body weight and body weight gain

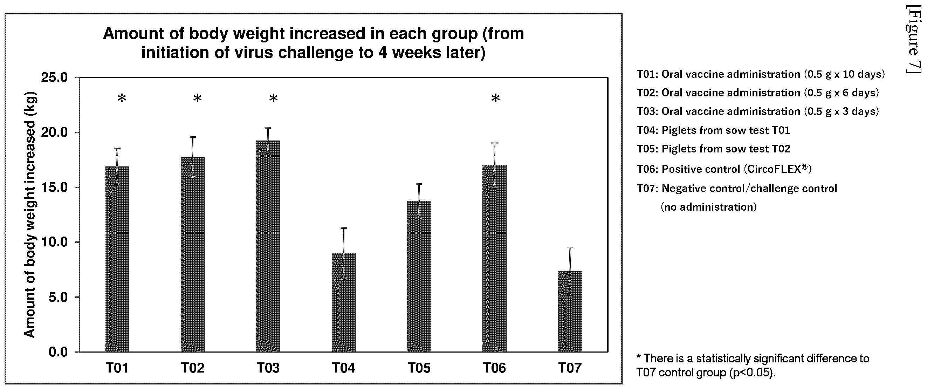

The average total body weight gain during the test period for 4 weeks was 7.3 kg in a no challenge administration control group (hereinafter referred to as T07), whereas it was 16.9 kg in a group administered with 0.5 g of Cap-expressing silkworm pupae for 10 days (hereinafter referred to as T01), was 17.8 kg in a group administered with 0.5 g of Cap-expressing silkworm pupae for 6 days (hereinafter referred to as T02), was 19.3 kg in a group administered with 0.5 g of Cap-expressing silkworm pupae for 3 days (hereinafter referred to as T03), was 9.0 kg in a piglet challenge group produced by administered sows (1 cycle) (hereinafter referred to as T04), was 13.8 kg in a piglet challenge group produced by administered sows (2 cycles) (hereinafter referred to as T05), and was 17.0 kg in a CircoFLEX administration group (one intramuscular administration) (hereinafter referred to as T6). Thus, the administration groups showed high body weight gain (Figure 7).

Among the administration groups, T01, T02, T03, T05, and T06 were confirmed to show high body weight gain. As a result of Dunnet's test (p<0.05), with regard to the total body gain (4-8W) during the test period for 4 weeks, T01, T02, T03 and T06 showed significantly higher numerical values than that of the negative control group T07 (Figure 7).

### 2) PCV2 viral gene quantification in blood

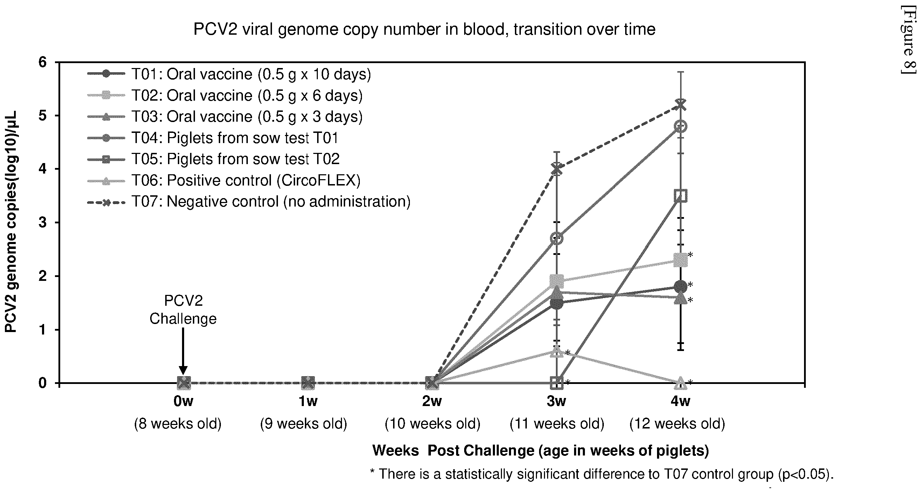

The average quantification numerical values of PCV2 genes in blood and the percentage to that of T07 (challenge control group) are shown in Table 6, and transitions in the gene levels over time are shown in Figure 8.

In the gene quantification, a high value (972125 copies/µL) was detected in T07 at 4 weeks after the challenge, while the percentage of each administration group to T07 was 0.4% in T01, 0.1% in T02, 0.3% in T03, 58.7% in T04, 39.0% in T05, and 0.0% in T06. Thus, in particular, in T01, T02, T03 and T06, the numerical values were significantly low.

In the measurement of the PCV2 gene level in blood, the lowest level was detected in the CircoFLEX administration group, followed by the 0.5 g of Cap-expressing silkworm pupae administration groups, which also showed a significantly lower level of detection, regardless of the dose.

**[Table 6]**

| Table 6 Comparison of PCV2 gene levels in blood and percentage of viral suppression against control group (T07) | | | | | |
|---|---|---|---|---|---|
| **Test group** | **Contents** | **Blood PCV2 (copies/µL)** | | **Percentage (%) to challenge control group (T07)(%)** | |
| | | **3W** | **4W** | **3W** | **4W** |
| **T01** | **Cap-expressing silkworm pupae 0.5 g x 10 days** | **1290** | **4371** | **12.9** | **0.4** |
| **T02** | **Cap-expressing silkworm pupae 0.5 g x 6 days** | **3668** | **1094** | **36.7** | **0.1** |
| **T03** | **Cap-expressing silkworm pupae 0.5 g x 3 days** | **1715** | **2601** | **17.1** | **0.3** |
| **T04** | **Piglets produced from administered sows (1 cycle)** | **848850** | **570275** | **8484.3** | **58.7** |
| **T05** | **Piglets produced from administered sows (2 cycles)** | **0 *** | **379388** | **0.0** | **39.0** |
| **T06** | **Positive control: Single intramuscular administration of CircoFLEX** | **57** | **0 *** | **0.6** | **0.0** |
| **T07** | **Negative control: Challenge control group, non-administration control** | **10005** | **972125** | **100.0** | **100.0** |

| | | | | | |
|---|---|---|---|---|---|
| Statistics: Dunnet *:P<0.05 | | | | | |

### 3) PCV2 antigen-specific antibody titer in serum

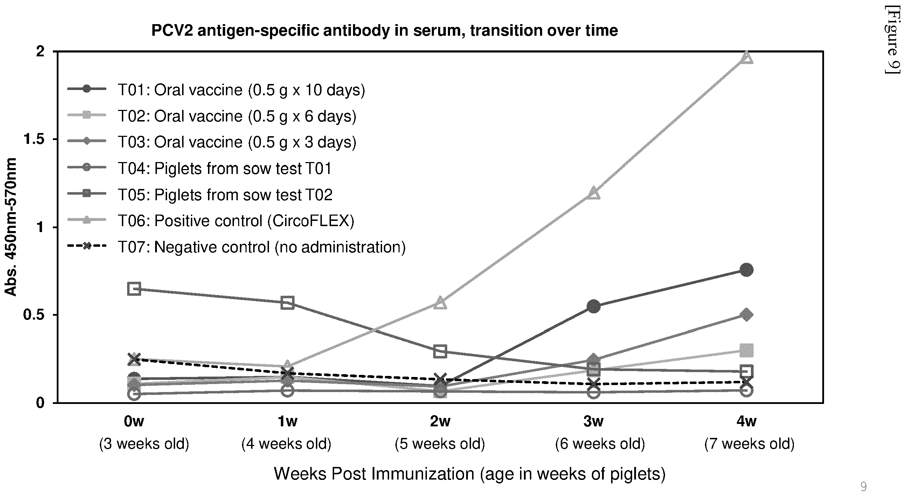

In order to evaluate the amount of antibody in serum, antigen-specific IgG was detected by using an ELISA method. As a result, in T06, the absorbance increased from the second week after the immunization, and at the fourth week after the immunization (before initiation of the challenge), the absorbance was found to be 1.969. In the oral immunization groups, the absorbance increased from the third week after the immunization, and at the fourth week after the immunization (before initiation of the challenge), the absorbance was found to be 0.3 to 0.757 (Figure 9).

### < Conclusion >

As a result of the virus challenge test, it was demonstrated that even if the dose and the days of administration were reduced (dosage and administration: 0.5 g of pupal powder x 3 days) in the oral immunization group to piglets, an increase in the body size, suppression of virus replication, and induction of an antigen-specific IgG antibody were observed, indicating that the vaccine had excellent effects against PCV2.

In addition, although transferred immunity alone did not show the effect of suppressing virus replication as much as the above-described oral immunization group, a certain multiplication effect was observed after the virus challenge. Thus, it was suggested that transferred immunity alone may be an effective means of immunization.

### [Example 4] Test of confirming disappearance of maternal antibody in piglets after oral administration of novel vaccine against PCV2 to pregnant pigs

### < Methods >

### 1. Test animals

Among piglets farrowed from four sows in each group, a total of sixty blood-collected piglets (5 piglets from a single sow) were subjected to the test.

### 2. Test category

The test category is shown in Table 7.

**[Table 7]**

| Test group | Test substance | Administration period to sows | Number of piglets |
|---|---|---|---|
| T01 | Novel vaccine | Approximately 8 and 4 weeks before expected farrowing | 20 piglets |
| T02 | Novel vaccine | Approximately 4 weeks before expected farrowing | 20 piglets |
| T03 | No administration | None | 20 piglets |

### 3. Test schedule

The test schedule is shown in Table 8.

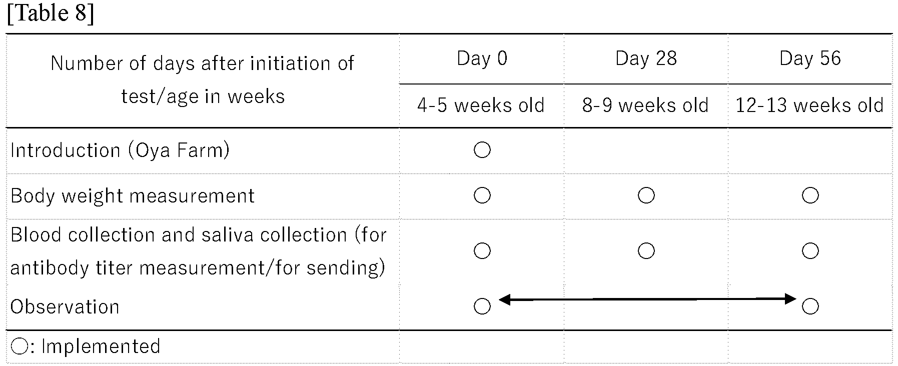

### 4. Observation items

1) Body weight measurement
   Individual piglets were weighed at Day 0 (4 to 5 weeks old), Day 28 (8 to 9 weeks old) and Day 56 (12 to 13 weeks old) after initiation of the test.
2) Blood and saliva collection
   Blood and saliva were collected from all animals on the day of the body weight measurement.
3) Clinical observation
   The general conditions of the animals (vitality, appetite, and fecal characteristics, etc.) were observed during the test period.
4) Antibody titer in blood
   IgG against PCV2, which was contained in the serum, was measured by an ELISA method.

### < Test results >

### 1. Body weight

The average body weights in individual groups were, in the order of 0 day, 28 days, and 56 days after initiation of the test were 16.5, 33.8 and 54.2 kg in T01; 10.3, 26.1 and 46.2 kg in T02; and 15.4, 33.7 and 54.1 kg in T03, respectively.

The average periodic body weight gains were, in the order of 0 to 28 days, 28 to 56 days, and the total period after initiation of the test, 17.3, 20.4 and 37.7 kg in T01; 15.9, 20.1 and 36.0 kg in T02; and 18.3, 20.4 and 38.7 kg in T03, respectively.

### 2. IgG antibody titer in serum

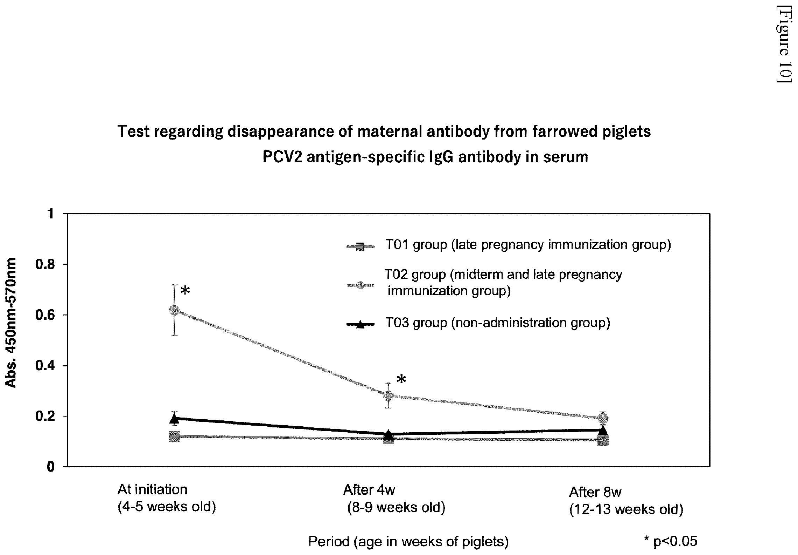

At initiation of the test (piglets with 4 to 5 weeks old) and at 4 weeks after initiation of the test (piglets with 8 to 9 weeks old), the absorbance of the T02 group was significantly higher than those of the other two groups. At 8 weeks after initiation of the test (piglets with 12 to 13 weeks old), the absorbance of the T02 group tended to be higher than those of the other two groups (Figure 10).

### < Conclusion >

As a result of the evaluation of the antibody disappearance of the maternal antibodies by the ELISA method, it was demonstrated that the piglets retained significantly more antibodies than the non-administration group even at the age of 8 to 9 weeks. Since some commercial injectable vaccines claim to be effective in maintaining maternal antibodies in piglets up to 5 weeks of age, it was considered that oral administration of silkworm-derived antigens to sows in the sow immunization test of the present Example provides excellent immunizing effects of the maternal antibodies to piglets.

### Sequence Listing Free Text

SEQ ID No: 1: Synthetic DNA
SEQ ID No: 2: Synthetic construct

## Claims

1. An oral vaccine composition against porcine circovirus associated diseases, comprising the pupa or cell of a baculovirus infectious insect, which is subjected to an infection treatment with a recombinant baculovirus, into which porcine circovirus type 2 protein-encoding DNA is introduced, and a freeze-drying treatment.

2. The oral vaccine composition according to claim 1, wherein the porcine circovirus type 2 protein-encoding DNA is as set forth in SEQ ID No: 1.

3. The oral vaccine composition according to claim 1, further comprising a solution containing an adjuvant.

4. The oral vaccine composition according to claim 1, which comprises at least 20% by weight of the pupa with respect to the total weight of the composition.

5. The oral vaccine composition according to claim 1, wherein the porcine circovirus associated disease is at least one selected from the group consisting of postweaning multisystemic wasting syndrome, porcine dermatitis and nephropathy syndrome, porcine respiratory disease complex, and reproductive disorder.

6. A method for producing an oral vaccine against porcine circovirus associated diseases, comprising a step of allowing a recombinant baculovirus, into which porcine circovirus type 2 protein-encoding DNA is introduced, to infect into the larva or pupa of a baculovirus infectious insect, and freeze-drying a pupa formed from the larva after the infection, or the pupa after the infection.

7. A method for producing an oral vaccine against porcine circovirus associated diseases, comprising a step of allowing a recombinant baculovirus, into which porcine circovirus type 2 protein-encoding DNA is introduced, to infect into baculovirus infectious cells, and freeze-drying the cells after the infection.

8. The oral vaccine composition according to claim 6 or 7, wherein the porcine circovirus type 2 protein-encoding DNA is as set forth in SEQ ID No: 1.

9. The method according to claim 6, wherein the insect is a silkworm.

10. The method according to claim 7, wherein the cells are derived from *Spilosoma imparilis, Antheraea pernyi, Bombyx mori* (silkworm), *Spodoptera frugiperda,* or *Trichoplusia ni.*

11. A method for immunizing a pig against a porcine circovirus, comprising a step of administering the composition according to any one of claims 1 to 5 to the pig.
